# FASCICULE DE BREVET EUROPEEN

(11) **EP 1 397 345 B1**
(45) Date de publication et mention de la délivrance du brevet: **28.11.2007**
(21) Numéro de dépôt: 02780840.1
(22) Date de dépôt: 13.06.2002
(51) Int. Cl.: C07C 253/30

(54) **PROCEDE D'HEMIHYDROGENATION DE DINITRILES EN AMINONITRILES**
VERFAHREN ZUR HEMIHYDRIERUNG VON DINITRILEN ZUR BILDUNG VON AMINONITRILEN
METHOD FOR THE HEMIHYDROGENATION OF DINITRILES IN ORDER TO FORM AMINONITRILES

(30) Priorité: 22.06.2001 FR 0108248
(43) Date de publication de la demande: 17.03.2004
(73) Titulaire: RHODIA POLYAMIDE INTERMEDIATES, 69192 Saint-Fons (FR)
(72) Inventeur: LECONTE, Philippe, F-69330 Meyzieu (FR); LOPEZ, Joseph, F-69100 Villeurbanne (FR); MARION, Philippe, F-69390 Vernaison (FR)
(74) Mandataire: Esson, Jean-Pierre
(86) Numéro de dépôt international: PCT/FR2002/002019
(87) Numéro de publication internationale: WO 2003/000650

(56) Documents cités:
- FR-A- 2 785 608
- US-A- 5 527 946
- US-A- 6 114 567
- US-A- 6 156 694

## Description

La présente invention concerne l'hémihydrogénation de dinitriles en aminonitriles correspondants.

Généralement l'hydrogénation des dinitriles est réalisée pour préparer les diamines correspondantes ; ainsi particulièrement l'hydrogénation de l'adiponitrile conduit à l'hexaméthylène diamine, elle-même étant l'un des deux composés de base de la préparation du polyamide-6,6.

Cependant, il peut s'avérer nécessaire de préparer non la diamine, mais l'aminonitrile intermédiaire. C'est par exemple, mais non limitativement le cas pour l'hémihydrogénation de l'adiponitrile en aminocapronitrile, susceptible d'être ensuite transformé en caprolactame composé de base du polyamide-6 ou directement en polyamide-6.

Ainsi le brevet US 4 389 348 décrit un procédé d'hydrogénation de dinitrile en oméga-aminonitrile, par l'hydrogène, en milieu solvant aprotique et ammoniac et en présence de rhodium déposé sur un support basique.

Le brevet US 5 151 543 décrit un procédé d'hydrogénation partielle de dinitriles en aminonitriles dans un solvant en excès molaire d'au moins 2/1 par rapport au dinitrile, comprenant de l'ammoniac liquide ou un alcanol contenant une base minérale soluble dans ledit alcanol, en présence d'un catalyseur de type nickel ou cobalt de Raney.

Le brevet US 5 981 790 concerne un procédé d'hydrogénation partielle de dinitriles en aminonitriles en présence d'un catalyseur à base de nickel de Raney ou cobalt de Raney en présence d'au moins 0,5 % en poids d'eau dans le milieu réactionnel contenant les produits à hydrogéner et les composés hydrogénés. Le catalyseur est utilisé conjointement avec une base.

Ces différents procédés permettent de produire conjointement un aminonitrile et une diamine dans des rapports plus ou moins différents et avec production plus ou moins importante de sous-produits difficiles à séparer. Des recherches permanentes sont entreprises pour modifier ce rapport pour notamment augmenter la production en aminonitrile au détriment de celle en diamine et également diminuer la formation de sous-produits.

Ainsi, la demande de brevet WO 00/64862 décrit un procédé d'hydrogénation partielle d'un dinitrile pour la production d'aminonitriles en présence d'un catalyseur d'hydrogénation, d'un solvant ammoniac liquide ou d'un alcanol et d'un composé permettant d'améliorer la sélectivité de la réaction en aminonitriles.

Il a également été proposé des systèmes catalytiques à base de nickel dopé par un ou plusieurs autres éléments métalliques comme le titane, le cuivre ou le fer pour améliorer l'activité du catalyseur, sa sélectivité en aminonitrile ou diminuer le taux de sous-produits indésirables. De tels systèmes catalytiques sont décrits dans les brevets FR2 785 608, US5 801 286.

Un des objectifs de la présente invention est de proposer un procédé d'hydrogénation préférentielle d'une seule fonction nitrile d'un dinitrile (appelée dans le présent texte hémihydrogénation) de manière à préparer majoritairement l'aminonitrile correspondant et seulement de manière minoritaire la diamine, avec formation minimale de sous-produits. Plus précisément l'invention concerne un procédé d'hémihydrogénation de dinitriles aliphatiques en aminonitriles correspondants, à l'aide d'hydrogène et d'un catalyseur d'hydrogénation. Ce procédé se caractérise en ce que le catalyseur d'hydrogénation comprend du nickel ou du nickel de Raney et un élément dopant choisi parmi le rhodium ou l'iridium.

Selon une autre caractéristique de l'invention, le catalyseur de l'invention peut comprendre un ou plusieurs dopants complémentaires choisis dans les groupes 3 à 12 de la classification périodique des éléments (selon la nomenclature IUPAC employée dans : Handbook of Chemistry and Physics - 80th edition 1999-2000). Notamment le groupe comprenant titane, chrome, fer, zirconium, vanadium, manganèse, bismuth, tantale, ruthénium, platine, palladium, niobium, hafnium, le bismuth et les éléments des terres rares sont préférées. Le catalyseur de l'invention ne peut contenir du cuivre, de l'argent et/ou de l'or.

Dans un mode de réalisation préféré de l'invention, une base minérale forte dérivant d'un métal alcalin ou alcalino-terreux ou ammonium est ajoutée dans le milieu d'hydrogénation. Toutefois, quand l'hydrogénation est réalisée en présence d'ammoniac liquide, la base minérale forte n'est pas obligatoirement nécessaire.

Selon une autre caractéristique préférentielle de l'invention, le milieu initial d'hydrogénation comporte de l'eau à raison d'au moins 0,5 % en poids par rapport à la totalité des composés liquides dudit milieu, de la diamine et/ou de l'aminonitrile susceptibles de se former à partir du dinitrile à hydrogéner ainsi que du dinitrile non transformé à raison pour l'ensemble de ces trois composés de 80 % à 99,5 %.

Dans un autre mode de réalisation du procédé de l'invention, la réaction peut être réalisée en présence d'un solvant tel qu'un alcool. Dans ce cas, la présence d'eau n'est pas obligatoire.

La réaction d'hémihydrogénation peut être réalisée en présence d'un additif augmentant la sélectivité en aminonitrile par rapport à celle obtenue avec le système décrit ci-dessus sans additif, tout en maintenant la sélectivité totale en aminonitrile et diamine à un niveau au moins sensiblement équivalent à celui obtenu sans l'additif. De tels additifs sont notamment décrits dans la demande de brevet WO 00/64862.
Selon l'invention, l'élément dopant rhodium ou iridium est présent dans le catalyseur, avantageusement, selon un rapport pondéral (Rh ou Ir)/Ni compris entre 0,05 % et 10 % et de préférence entre 0,1 % et 5 %.

La quantité de dopant complémentaire autre que le rhodium ou l'iridium que peut contenir le catalyseur représente généralement de 0 % à 5 % en poids du poids du nickel.

Le catalyseur est préparé selon les méthodes classiques de préparation des catalyseurs métalliques.

Ainsi, à titre d'exemple, les nickels de RANEY sont des catalyseurs largement utilisés dans l'industrie pour les réactions d'hydrogénation. Ils sont préparés par attaque alcaline d'alliages Al/Ni riches en aluminium et contenant le cas échéant d'autres métaux, généralement appelés dopants ou promoteurs. Le catalyseur est constitué par des agglomérats de cristallites de nickel à grande surface spécifique et à concentration en aluminium résiduel variable. Le dopant peut être ajouté à différentes étapes de la préparation du catalyseur : lors de la préparation de l'alliage par fusion des différents métaux, lors de l'activation de l'alliage par attaque alcaline en présence de sel de Rh ou d'Ir ou après activation de l'alliage (cf. brevets WO 95/17959 et WO 95/17960).

Ce catalyseur comprend généralement une teneur en aluminium, exprimée en poids par rapport au poids du nickel inférieure ou égale à 10 %.

Le catalyseur de l'invention peut être mis en oeuvre sous différentes formes tels que grains, granulés, poudre.

Le procédé de l'invention permet d'obtenir, pour un taux de transformation du dinitrile supérieur à 70 %, une sélectivité en aminonitrile supérieure à 55 % et une sélectivité totale en aminonitrite et diamine supérieure à 90 %.

Les dinitriles aliphatiques qui peuvent être mis en oeuvre dans le procédé de l'invention sont plus particulièrement les dinitriles de formule générale (I):

NC-R-CN (I)

dans laquelle R représente un groupement alkylène ou alcénylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié ayant de 2 à 6 atomes de carbone.

A titre d'exemples de tels dinitriles, on peut citer notamment l'adiponitrile, le méthylglutaronitrile, l'éthylsuccinonitrile, le malononitrile, le succinonitrile, le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

En pratique, le cas où R = (CH₂)₄ sera le plus fréquent car cela correspond à la mise en oeuvre de l'adiponitrile (ADN) dans le présent procédé.

La base minérale forte est généralement constituée par les hydroxydes, les carbonates et les alcanolates de métal alcalin ou de métal alcalino-terreux ou d'ammonium. Elle est choisie de préférence parmi les hydroxydes, les carbonates et les alcanolates de métal alcalin ou d'ammonium.

De façon privilégiée, la base minérale forte mise en oeuvre est choisie parmi les composés suivants : LiOH, NaOH, KOH, RbOH, CsOH, NH4OH et leur mélanges.

En pratique, on utilise le plus souvent NaOH et KOH bien que RbOH et CsOH puissent donner de très bons résultats.

Le milieu réactionnel a une composition variant selon le type de mise en oeuvre du procédé.

L'eau est habituellement présente dans le milieu réactionnel dans une quantité inférieure ou égale à 20 % en poids. Préférentiellement, la teneur en eau du milieu réactionnel est comprise entre 2 % et 15 % en poids par rapport à l'ensemble des constituants liquides dudit milieu.

En complément ou en substitution à l'eau, on peut prévoir au moins un autre solvant, généralement de type alcool. Les alcools qui conviennent plus particulièrement sont par exemple le méthanol, l'éthanol, le propanol, l'isopropanol, le butanol et les mélanges desdits composés.

Lorsqu'il est employé avec l'eau, le solvant alcoolique représente de deux à quatre parties en poids pour une partie en poids d'eau et de préférence trois parties pour une partie d'eau.

Selon une autre caractéristique préférée de l'invention, le milieu initial d'hydrogénation comprend de la diamine qui est coproduite par l'hydrogénation. Il s'agit par exemple d'hexaméthylènediamine lorsque le substrat dinitrile est l'adiponitrile.

La concentration moyenne en régime continu de l'aminonitrile et/ou de la diamine dans le milieu réactionnel est avantageusement comprise entre 35 % et 99 % en poids par rapport au poids de la totalité du solvant inclus dans ledit milieu réactionnel et, plus préférentiellement entre 45 % et 89 % en poids par poids.

La concentration moyenne du milieu réactionnel en dinitrile se situe entre 10 % et 45 % en poids par poids, quand le procédé est mis en oeuvre de façon continue.

La concentration totale en aminonitrile visé et/ou de la diamine correspondante et du dinitrile non transformé dans le milieu réactionnel est généralement comprise entre 85 % et 99 % en poids par rapport à l'ensemble des liquides inclus dans ledit milieu réactionnel.

Le milieu réactionnel peut comporter de l'ammoniac liquide ou dissous. Généralement l'ammoniac représente de 0 % à 50 % en poids du milieu réactionnel et de préférence de 0 %à15%.

La quantité d'hydroxyde alcalin ou alcalino-terreux ou d'ammonium dans le milieu réactionnel varie en fonction de la nature dudit milieu réactionnel.

Dès lors que le milieu réactionnel ne contient que de l'eau, les produits de la réaction et éventuellement de l'ammoniac ou la diamine, à titre de milieu solvant liquide, la quantité d'hydroxyde de métal alcalin ou alcalino-terreux est avantageusement supérieure ou égale à 0,1 mot/kg de catalyseur, de préférence comprise entre 0,1 et 2 mol/kg de catalyseur et plus préférentiellement encore entre 0,2 et 1,0 mol/kg de catalyseur.

La quantité de catalyseur mise en oeuvre peut varier très largement en fonction notamment du mode de fonctionnement adopté ou des conditions réactionnelles choisies. Ainsi, si l'on introduit progressivement le dinitrile dans le milieu réactionnel, le rapport pondéral catalyseur/dinitrile à hydrogéner sera beaucoup plus élevé que si tout le dinitrile est mis en oeuvre dès le début de la réaction. A titre indicatif, on peut utiliser de 0,5 % à 50 % en poids de catalyseur par rapport au poids total du milieu réactionnel et le plus souvent de 1 % à 35 %.

Selon un mode de réalisation préféré de l'invention, le catalyseur est préconditionné avant son introduction dans le milieu d'hémihydrogénation. Ce préconditionnement est avantageusement réalisé selon le procédé décrit dans la demande de brevet français non publiée n° 00.02997. Ce procédé consiste brièvement à mélanger le catalyseur d'hydrogénation avec une quantité déterminée de base minérale forte et un solvant dans lequel la base minérale forte est peu soluble. Selon l'invention, le milieu contenant le catalyseur ainsi conditionné est alimenté dans le réacteur d'hydrogénation, la réaction d'hydrogénation étant réalisée selon les conditions et procédures habituelles et déjà décrites dans la littérature.

La sélectivité en aminonitrile, à taux de transformation du dinitrile constant, dépend notamment de la nature et de la teneur en dopant, de la quantité d'eau dans le milieu réactionnel, de la nature de la base et du rapport base/Ni.

Le procédé de l'invention est généralement mis en oeuvre à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 1bar (0,10 MPa) et 100 bar (10 MPa) et de préférence entre 5 bar (0,5 MPa) et 50 bar (5 MPa).

La durée de la réaction est variable en fonction des conditions réactionnelles et du catalyseur.

Dans un mode de fonctionnement discontinu, elle peut varier de quelques minutes à plusieurs heures.

Il est à noter que l'homme du métier peut moduler la chronologie des étapes du procédé selon l'invention, selon les conditions opératoires.

Les autres conditions qui régissent l'hydrogénation (en mode continu ou discontinu) conforme à l'invention, relèvent de dispositions techniques traditionnelles et connues en elles-mêmes.

L'invention est illustrée par les exemples qui suivent d'hémihydrogénation d'adiponitrile en amino-6 capronitrile.

Dans ces exemples les abréviations suivantes pourront être utilisées :
- AdN = adiponitrile
- ACN = aminocapronitrile
- HMD = hexaméthylène diamine
- TT = taux de transformation
- RT = sélectivité par rapport au substrat de départ transformé (ici par rapport à l'ADN).

### EXEMPLE COMPARATIF

Dans un réacteur en acier inoxydable de 100 ml, équipé d'une agitation de type rushton autoaspirante, de moyens d'introduction des réactifs et de l'hydrogène et d'un système de régulation de température, on charge :
- hexaméthylène diamine 24 g
- eau 5,3 g
- KOH 0,33 mmol
- Ni de Raney (à 1,7 % de Cr) 0,65 g

Dans cet exemple il y a 0,5 mol KOH/kg Ni.

Après avoir purgé le réacteur à l'azote, puis à l'hydrogène ; la pression est alors réglée à 2 MPa d'hydrogène et le mélange réactionnel est chauffé à 50°C.
24 g d'adiponitrile sont ensuite introduits instantanément par l'intermédiaire d'une ampoule de coulée pressurisée à 2,5 Mpa par un manodétendeur placé sur une réserve d'hydrogène à 5 MPa. Le temps est pris égal à 0 à cet instant. L'avancement de la réaction est suivi par la consommation d'hydrogène dans la réserve d'hydrogène, la pression dans le réacteur étant maintenue constante à 2,5 Mpa, et par l'analyse par chromatographie en phase vapeur (CPG) d'un prélèvement du mélange réactionnel. Lorsque le maximum de rendement en aminocapronitrile est atteint, on arrête la réaction par arrêt de l'agitation, refroidissement du mélange réactionnel puis dépressurisation.

On obtient les résultats suivants :
- durée de la réaction : 33 min
- TT de l'AdN : 79,6 %
- RT en ACN : 70,1 %
- RTenHMD : 29,5%
- RT en autres produits : 0,4 %

### EXEMPLE 1 : Ni dopé Rh

Dans un réacteur en acier inoxydable de 100 ml, équipé d'une agitation de type rushton autoaspirante, de moyens d'introduction des réactifs et de l'hydrogène et d'un système de régulation de température, on charge :
- hexaméthylène diamine 24 g
- eau 5,3 g
- KOH 0,66 mmol
- Ni de Raney dopé à 2,7 % de Rh 1,3 g

Dans cet exemple il y a 0,5 mol KOH/kg Ni.

Après avoir purgé le réacteur à l'azote, puis à l'hydrogène ; la pression est alors réglée à 2 MPa d'hydrogène et on chauffe le mélange réactionnel à 50°C.
24 g d'adiponitrile sont ensuite introduits instantanément par l'intermédiaire d'une ampoule de coulée pressurisée à 2,5 Mpa par un manodétendeur placé sur une réserve d'hydrogène à 5 MPa. Le temps est pris égal à 0 à cet instant. L'avancement de la réaction est suivi par la consommation d'hydrogène dans la réserve, la pression dans le réacteur étant maintenue constante à 2,5 Mpa, et par l'analyse par chromatographie en phase vapeur (CPG) d'un prélèvement du mélange réactionnel. Lorsque l'optimum de rendement en aminocapronitrile est atteint, on arrête la réaction par arrêt de l'agitation, refroidissement du mélange réactionnel puis dépressurisation.

On obtient les résultats suivants :
- durée de la réaction : 66 min
- TT de l'AdN : 83,3 %
- RTenACN : 71,7%
- RT en HMD : 28 %
- RT en autres produits : 0,3 %

### EXEMPLE 2 : Ni dopé Ir

Dans un réacteur en acier inoxydable de 100 ml, équipé d'une agitation de type rushton autoaspirante, de moyens d'introduction des réactifs et de l'hydrogène et d'un système de régulation de température, on charge :
- hexaméthylène diamine 24 g
- eau 5,3 g
- KOH 0,13 mmol
- Ni de Raney dopé à 2,8 % de Ir 1,3 g

Dans cet exemple il y a 0,2 mol KOH/kg Ni.

Après avoir purgé le réacteur à l'azote, puis à l'hydrogène ; la pression est alors réglée à 2 MPa d'hydrogène et on chauffe le mélange réactionnel à 50°C.
24 g d'adiponitrile sont ensuite introduits instantanément par l'intermédiaire d'une ampoule de coulée pressurisée à 2,5 Mpa par un manodétendeur placé sur une réserve d'hydrogène à 5 MPa. Le temps est pris égal à 0 à cet instant. L'avancement de la réaction est suivi par la consommation d'hydrogène dans la réserve, la pression dans le réacteur étant maintenue constante à 2,5 Mpa, et par l'analyse par chromatographie en phase vapeur (CPG) d'un prélèvement du mélange réactionnel. Lorsque l'optimum de rendement en aminocapronitrile est atteint, on arrête la réaction par arrêt de l'agitation, refroidissement du mélange réactionnel puis dépressurisation.

On obtient les résultats suivants :
- durée de la réaction : 72 min
- TT de l'AdN : 81,2 %
- RT en ACN : 73,8 %
- RT en HMD : 25,4 %
- RT en autres produits : 0,8 %

## Revendications

1. Procédé d'hémihydrogénation d'un dinitrile en aminonitrile correspondant, dans un milieu liquide, **caractérisé en ce que** l'on opère en présence d'un catalyseur nickel ou nickel de Raney contenant un élément dopant parmi le rhodium ou l'iridium, et le catalyseur ne peut contenir du cuivre, de l'argent et/ou de l'or.

2. Procédé selon la revendication 1, **caractérisé en ce que** le rapport pondéral (Rh ou Ir)/Ni du catalyseur utilisé est compris entre 0,05 % et 10 %.

3. Procédé selon la revendication 2, **caractérisé en ce que** le rapport pondéral précité est compris entre 0,1 % et 5 % .

4. Procédé selon l'une des revendications 1 ou 2, **caractérisé en ce que** le catalyseur contient un ou plusieurs dopants complémentaires choisis parmi le titane, le chrome, le fer, le zirconium, le vanadium, le manganèse, le bismuth, le tantale, le ruthénium, le platine, le palladium, le niobium, l'hafnium, les éléments des terres rares, à l'exception du cuivre, de l'argent ou de l'or.

5. Procédé selon la revendication 4, **caractérisé en ce que** la quantité de dopant complémentaire autre que le rhodium ou l'iridium que contient le catalyseur représente de 0 % à 5 % en poids du poids du nickel.

6. Procédé selon l'une des revendications précédentes **caractérisé en ce qu'**il est mis en oeuvre en présence d'une base forte.

7. Procédé selon l'une des revendications 1 à 6, **caractérisé en ce qu'**il s'applique aux substrats dinitriles de formule (I):
NC-R-CN (I)
dans laquelle R représente un groupement alkylène, linéaire ou ramifié, ayant de 1 à 12 atomes de carbone et de préférence un radical alkylène, linéaire ou ramifié, ayant de de 1 à 6 atomes de carbone.

8. Procédé selon l'une des revendications 1 à 7, **caractérisé en ce que** la concentration moyenne du milieu réactionnel en dinitrile se situe entre 10 % et 45 % en poids par poids, quand le procédé est mis en oeuvre de façon continue.

9. Procédé selon l'une des revendications 1 à 8, **caractérisé en ce qu'**il est mis en oeuvre en présence de LiOH, NaOH, KOH, RbOH, CsOH ou NH₄OH ou de leurs mélanges.

10. Procédé selon l'une des revendications 1 à 9, **caractérisé en ce que** le milieu réactionnel liquide initial comprend de l'eau représentant une quantité inférieure ou égale à 20 % en poids par rapport au poids du milieu réactionnel total.

11. Procédé selon la revendication 10, **caractérisé en ce que** la concentration en eau est comprise entre 2 et 15 % en poids par poids du milieu réactionnel.

12. Procédé selon l'une des revendications 1 à 11, **caractérisé en ce que** le milieu réactionnel comporte un solvant de type alcool.

13. Procédé selon la revendication 12, **caractérisé en ce que** l'alcool est choisi parmi le méthanol, l'éthanol, le propanol, l'isopropanol, le butanol ou les mélanges desdits composés.

14. Procédé selon l'une des revendications 1 à 13, **caractérisé en ce que** le milieu réactionnel initial comporte de la diamine qui est coproduite par l'hydrogénation.

15. Procédé selon la revendication 14, **caractérisé en ce que** la concentration moyenne en régime continu de l'aminonitrile et/ou de la diamine dans le milieu réactionnel est comprise entre 35 % et 99 % en poids par rapport au poids de la totalité du solvant inclus dans ledit milieu réactionnel.

16. Procédé selon la revendication 15, **caractérisé en ce que** la concentration moyenne en aminonitrile et/ou de la diamine est comprise entre 45 % et 89 % en poids par poids de la totalité du solvant dans le milieu réactionnel.

17. Procédé selon l'une des revendications 1 à 16, **caractérisé en ce que** le milieu réactionnel comporte l'ammoniac liquide ou dissous.

18. Procédé selon la revendication 17, **caractérisé en ce que** l'ammoniac représente de 0 % à 50 % en poids du milieu réactionnel.

19. Procédé selon l'une des revendications 6 à 18, **caractérisé en ce que** la quantité d'hydroxyde de métal alcalin ou alcalino-terreux ou ammoniaque est supérieure ou égale à 0,1 mol/kg de catalyseur.

20. Procédé selon la revendication 19, **caractérisé en ce que** la quantité d'hydroxyde précitée est comprise entre 0,1 et 2 mol/kg de catalyseur.

21. Procédé selon la revendication 19 ou 20, **caractérisé en ce que** la quantité d'hydroxyde précitée est comprise entre 0,2 et 1,0 mol/kg de catalyseur.

22. Procédé selon l'une des revendications 1 à 21, **caractérisé en ce qu'**il est réalisé à une température inférieure ou égale à 150°C, de préférence inférieure ou égale à 120°C.

23. Procédé selon l'une des revendications 1 à 22, **caractérisé en ce qu'**il est réalisé sous une pression en hydrogène comprise entre 0,10 et 10 MPa.

## Claims

1. Process for the hemihydrogenation of a dinitrile to corresponding aminonitrile in a liquid medium, **characterized in that** the process is carried out in the presence of a nickel or Raney nickel catalyst comprising a doping element chosen from rhodium or iridium, and the catalyst cannot comprise copper, silver and/or gold.

2. Process according to Claim 1, **characterized in that** the (Rh or Ir) /Ni ratio by weight of the catalyst used is between 0.05% and 10%.

3. Process according to Claim 2, **characterized in that** the abovementioned ratio by weight is between 0.1% and 5%.

4. Process according to either of Claims 1 and 2, **characterized in that** the catalyst comprises one or more additional dopants chosen from titanium, chromium, iron, zirconium, vanadium, manganese, bismuth, tantalum, ruthenium, platinum, palladium, niobium, hafnium or the rare earth metal elements, with the exception of copper, silver or gold.

5. Process according to Claim 4, **characterized in that** the amount of additional dopant other than rhodium or iridium which the catalyst comprises represents from 0% to 5% by weight of the weight of the nickel.

6. Process according to one of the preceding claims, **characterized in that** it is carried out in the presence of a strong base.

7. Process according to one of Claims 1 to 6, **characterized in that** it is applied to the dinitrile substrates of formula (I):
NC-R-CN (I)
in which R represents a linear or branched alkylene group having from 1 to 12 carbon atoms and preferably a linear or branched alkylene radical having from 1 to 6 carbon atoms.

8. Process according to one of Claims 1 to 7, **characterized in that** the mean concentration of dinitrile in the reaction medium lies between 10% and 45% as weight by weight, when the process is carried out continuously.

9. Process according to one of Claims 1 to 8, **characterized in that** it is carried out in the presence of LiOH, NaOH, KOH, RbOH, CsOH or NH₄OH or of their mixtures.

10. Process according to one of Claims 1 to 9, **characterized in that** the starting liquid reaction medium comprises water representing an amount less than or equal to 20% by weight with respect to the weight of the total reaction medium.

11. Process according to Claim 10, **characterized in that** the concentration of water is between 2 and 15% as weight by weight of the reaction medium.

12. Process according to one of Claims 1 to 11, **characterized in that** the reaction medium comprises a solvent of alcohol type.

13. Process according to Claim 12, **characterized in that** the alcohol is chosen from methanol, ethanol, propanol, isopropanol, butanol or the mixtures of the said compounds.

14. Process according to one of Claims 1 to 13, **characterized in that** the initial reaction medium comprises diamine which is coproduced by the hydrogenation.

15. Process according to Claim 14, **characterized in that** the mean concentration under continuous conditions of the aminonitrile and/or of the diamine in the reaction medium is between 35% and 99% by weight with respect to the weight of the combined solvent included in the said reaction medium.

16. Process according to Claim 15, **characterized in that** the mean concentration of the aminonitrile and/or of the diamine is between 45% and 89% as weight by weight of the combined solvent in the reaction medium.

17. Process according to one of Claims 1 to 16, **characterized in that** the reaction medium comprises liquid or dissolved ammonia.

18. Process according to Claim 17, **characterized in that** the ammonia represents from 0% to 50% by weight of the reaction medium.

19. Process according to one of Claims 6 to 18, **characterized in that** the amount of alkali metal or alkaline earth metal hydroxide or ammonia is greater than or equal to 0.1 mol/kg of catalyst.

20. Process according to Claim 19, **characterized in that** the abovementioned amount of hydroxide is between 0.1 and 2 mol/kg of catalyst.

21. Process according to Claim 19 or 20, **characterized in that** the abovementioned amount of hydroxide is between 0.2 and 1.0 mol/kg of catalyst.

22. Process according to one of Claims 1 to 21, **characterized in that** it is carried out at a temperature of less than or equal to 150°C, preferably of less than or equal to 120°C.

23. Process according to one of Claims 1 to 22, **characterized in that** it is carried out under a hydrogen pressure of between 0.10 and 10 MPa.

## Patentansprüche

1. Verfahren zur Halbhydrierung eines Dinitrils in das entsprechende Aminonitril in einem flüssigen Medium, **dadurch gekennzeichnet, dass** in Gegenwart eines Nickel- oder Raney-Nickelkatalysators gearbeitet wird, der ein Dotierungselement aus Rhodium oder Iridium enthält, und dass der Katalysator kein Kupfer, Silber und/oder Gold enthalten darf.

2. Verfahren nach Anspruch 1, **dadurch gekennzeichnet, dass** das Gewichtsverhältnis (Rh oder Ir)/Ni des verwendeten Katalysators 0,05 % bis 10 % beträgt.

3. Verfahren nach Anspruch 2, **dadurch gekennzeichnet, dass** das zuvor genannte Gewichtsverhältnis 0,1 % bis 5 % beträgt.

4. Verfahren nach einem der Ansprüche 1 oder 2, **dadurch gekennzeichnet, dass** der Katalysator ein oder mehrere zusätzliche Dotierungsmittel enthält, die aus Titan, Chrom, Eisen, Zirkonium, Vanadium, Mangan. Wismut, Tantal, Ruthenium, Platin, Palladium, Niob, Hafnium, den Elementen der Seltenen Erden, mit Ausnahme von Kupfer, Silber oder Gold ausgewählt sind.

5. Verfahren nach Anspruch 4, **dadurch gekennzeichnet, dass** die Menge an zusätzlichem Dotierungsmittel, das anders ist als Rhodium oder Iridium, das der Katalysator enthält, 0 bis 5 Gew.-% des Gewichts des Nickels ausmacht.

6. Verfahren nach einem der vorhergehenden Ansprüche, **dadurch gekennzeichnet, dass** es in Gegenwart einer starken Base durchgeführt wird.

7. Verfahren nach einem der Ansprüche 1 bis 6, **dadurch gekennzeichnet, dass** es auf Dinitrilsubstrate der Formel (I):
NC-R-CN (I)
zutrifft, wobei R eine lineare oder verzweigte Alkylengruppe mit 1 bis 12 Kohlenstoffatomen und vorzugsweise einen linearen oder verzweigten Alkylenrest mit 1 bis 6 Kohlenstoffatomen darstellt.

8. Verfahren nach einem der Ansprüche 1 bis 7, **dadurch gekennzeichnet, dass** die durchschnittliche Konzentration des Reaktionsmediums an Dinitril zwischen 10 und 45 Gew.-% des Gewichts des Reaktionsmediums liegt, wenn das Verfahren kontinuierlich durchgeführt wird.

9. Verfahren nach einem der Ansprüche 1 bis 8, **dadurch gekennzeichnet, dass** es in Gegenwart von LiOH, NaOH, KOH, RbOH, CsOH oder NH₄OH oder ihren Gemischen durchgeführt wird.

10. Verfahren nach einem der Ansprüche 1 bis 9, **dadurch gekennzeichnet, dass** das flüssige Reaktions-Ausgangsmedium Wasser einschließt, das eine Menge von kleiner oder gleich 20 Gew.-%, bezogen auf das Gewicht des gesamten Reaktionsmediums, ausmacht.

11. Verfahren nach Anspruch 10, **dadurch gekennzeichnet, dass** die Konzentration an Wasser 2 bis 15 Gew.-% des Gewichts des Reaktionsmediums beträgt.

12. Verfahren nach einem der Ansprüche 1 bis 11, **dadurch gekennzeichnet, dass** das Reaktionsmedium ein alkoholisches Lösungsmittel umfasst.

13. Verfahren nach Anspruch 12, **dadurch gekennzeichnet, dass** der Alkohol aus Methanol, Ethanol, Propanol, Isopropanol, Butanol oder den Gemischen der Verbindungen ausgewählt ist.

14. Verfahren nach einem der Ansprüche 1 bis 13, **dadurch gekennzeichnet, dass** das Reaktionsmedium das Diamin einschließt, das durch die Hydrierung mitproduziert wird.

15. Verfahren nach Anspruch 14, **dadurch gekennzeichnet, dass** die durchschnittliche Konzentration bei kontinuierlichem Betrieb des Aminonitrils und/oder des Diamins in dem Reaktionsmedium 35 bis 99 Gew.-% bezüglich des Gewichts der Gesamtheit des Lösungsmittels, das in dem Reaktionsmedium eingeschlossen ist, beträgt.

16. Verfahren nach Anspruch 15, **dadurch gekennzeichnet, dass** die durchschnittliche Konzentration an Aminonitril und/oder des Diamins 45 bis 89 Gew.-% des Gewichts der Gesamtheit des Lösungsmittels in dem Reaktionsmedium beträgt.

17. Verfahren nach einem der Ansprüche 1 bis 16, **dadurch gekennzeichnet, dass** das Reaktionsmedium flüssigen oder gelösten Ammoniak umfasst.

18. Verfahren nach Anspruch 17, **dadurch gekennzeichnet, dass** der Ammoniak 0 bis 50 Gew.-% des Reaktionsmediums darstellt.

19. Verfahren nach einem der Ansprüche 6 bis 18, **dadurch gekennzeichnet, dass** die Menge an Alkali- oder Erdalkalimetallhydroxid oder Ammoniak größer oder gleich 0,1 mol/kg Katalysator ist.

20. Verfahren nach Anspruch 19, **dadurch gekennzeichnet, dass** die zuvor genannte Menge an Hydroxid 0,1 bis 2 mol/kg Katalysator beträgt.

21. Verfahren nach Anspruch 19 oder 20, **dadurch gekennzeichnet, dass** die zuvor genannte Menge an Hydroxid 0,2 bis 1,0 mol/kg Katalysator beträgt.

22. Verfahren nach einem der Ansprüche 1 bis 21, **dadurch gekennzeichnet, dass** es bei einer Temperatur von kleiner oder gleich 150 °C, vorzugsweise von kleiner oder gleich 120 °C, durchgeführt wird.

23. Verfahren nach einem der Ansprüche 1 bis 22, **dadurch gekennzeichnet, dass** es unter einem Wasserstoffdruck von 0,10 bis 10 MPa durchgeführt wird.
